# EUROPEAN PATENT APPLICATION

(11) **EP 2 757 098 A1**
(43) Date of publication of application: **23.07.2014**
(21) Application number: 13151702.1
(22) Date of filing: 17.01.2013
(51) Int. Cl.: C07D 249/04, A61L 27/00, C07K 7/00

(54) **Compounds for Functionalizing Biomaterials**

(71) Applicant: Ecole Polytechnique Fédérale de Lausanne (EPFL), 1015 Lausanne (CH)
(72) Inventor: Gerber, Sandrine, 1163 Etoy (CH); Borcard, Françoise, 1627 Vaulruz (CH); Juillerat, Lucienne, 1000 Lausanne 26 (CH); Gonzenbach, Urs, 8107 Buchs (CH); Krauss Juillerat, Franziska, 7417 Paspels (CH)
(74) Representative: Schneiter, Sorin

(57) **Abstract**

The present invention provides compounds suitable for functionalizing biomaterials, in particular prosthetics and implant materials, functionalized biomaterials, and methods for synthesizing the compound. The compounds contain an anchoring group, a group for covalent binding to modified osteoblast precursor cells and a group for binding to endothelial cells or precursor cells.

## Description

### Technical Field

The present invention relates to the field of prosthetics, biomaterials and implant materials, in particular bone implant materials. More specifically, the invention relates to novel biomaterials and implant materials, to compounds that can be used for functionalization of biomaterials and implants materials, to various methods of using the compounds of the invention, and to compounds for use as starting material and/or for synthesizing compounds.

### Prior Art and the Problem Underlying the Invention

Over the last decade, bone related diseases such as osteogenesis imperfecta, osteoarthritis, osteomyelitis, and osteoporosis, traumatic injuries and orthopedic surgeries have dramatically increased. Reasons for this drastic development can be found in the ageing of the population and the democratization of high-risk sports. For example, the number of total joint arthroplasties and revision surgeries in the US has increased from 700'000 in 1998 to over 1.1 million in 2005. Medical expenses related to fracture, reattachment, and replacement of hip and knee joints were estimated to be over $20 billion in 2003, and predicted to increase to over $74 billion by the year 2015. Spinal arthrodesis is another example of a surgery typically requiring substantial bone repair/replacement. Since 1997, spinal arthrodesis has seen the highest percentage of increase for all muscoskeletal surgeries requiring hospitalization. In 2005 alone, over 300'000 spinal fusions were conducted costing over $20 billion. Traumatic bone fractures accounted for 8.5 million physicians visits, almost 1 million of which required hospitalization in 2005. Also, in 2005, there were over 3000 pediatric hospitalizations for bone cancer costing over $70 million.

There exist various ways of treating tissue defects in the art. For treating bone defects, for example, today's gold standard is still autologous iliac crest bone grafting.

There are advantages but also several important inconveniences associated with the removal of bone tissue from a patient or from a donor for grafting it to damaged bone. Success in both allografts and autografts are dependent on the physical and biological similarity between the donor and host tissue. One inconvenience is that removal of bone from a subject is itself an invasive procedure, resulting in an increased risk of infections, pain, morbidity, the risk of disease transmission, and rejection. Furthermore, bone removal inevitably weakens bone structure at the site of removal, which may be a severe problem in patients suffering from osteoporosis, for example, or can transmit defective bone tissue for example in osteoporosis or cancer. Moreover, the availability of healthy bone substance in a subject is limited, and may not suffice to accommodate needs in case of extensive defects and/or for treating defects repeatedly and at different locations, for example. Similar problems exist in the case of other tissue materials.

There is thus a need for providing artificial or synthetic biomaterials, implant materials and prosthetics. While such materials have been suggested, they may suffer from low biocompatibility. In many cases, the understanding of the biology and physiology of the bone is insufficient and does not mimic the bone structure and functions as close as possible.

Therefore, the present inventors have found that it may be necessary to provide ways for promoting efficient tissue regeneration within the biomaterial. A close interaction and continuous interface between the biomaterial and natural tissue could be advantageous for achieving a functional implant and efficient tissue repair.

In accordance with the invention, it is an objective to provide biomaterials, implant materials and prosthetics for treating bone damages, in particular synthetic materials.

In particular, it is an objective to provide ways for promoting efficient tissue regeneration within biomaterials and implants. It is an objective of the invention to provide biomaterials that can be seeded with osteogenic cells or osteoinductive growth factors in general. It is an objective of the invention to provide a biomaterial that is functionalized so as to interact or bind to osteogenic cells, for example human fetal osteoblasts.

It is also an objective of the invention to promote vascularisation of a biomaterial that can be used for treating bone damages. It is an objective of the invention to provide biomaterials that promote the development of a vascular system on and/or even inside the biomaterial. It is an objective of the invention to provide a biomaterial that is functionalized so as to interact or bind to angiogenic factors and/or cells, for example endothelial cells or endothelial precursor cells.

H. Comas et al, ACS Appl. Mater. Interfaces 2012, 4, 573-573, disclose the functionalization of alumina ceramic foams with linker compounds. However, these linker compounds do not address the objectives set out above.

F. Borcard et al, Bioconjugate Chem. 2011, 22, 1422-1432 disclose compounds that contain a biotin marker moiety as well as a moiety containing an alkyne derivative. These marker compounds can be coupled to cells expressing azido-modified proteins by way of a copper-mediated or copper-free click reaction. This reference does not address the objectives put forward above.

F. Borcard et al, J. Med. Chem. 2012, 55 (18) 1988-7997 disclose a multifunctional ligand containing a cyclic arginine-glycine-aspartate (RGD) peptide, a tetraethylene glycol spacer and a gallate moiety. The binding of this ligand to dense bioceramics and its ability to increase the endothelial cell adhesion were demonstrated. However, for binding this multifunctional ligand to cells expressing azido-modified proteins, a copper catalyst is required, which is disadvantageous due to high risk of cytotoxicity. Furthermore, the yields of the synthesis of this ligand are not satisfactory. It is thus an objective to provide a ligand or linker compound that can be produced in high yields and which can effectively be bound to cells under mild, physiological conditions.

In view of the above, it is an objective to provide a linker or ligand compound that can be used to functionalize biomaterials, which is biocompatible and well tolerated in a patient's body, which can be synthesized rapidly and in high yields on the basis of available starting materials, and which is capable of promoting tissue regeneration and vascularization of implants and biomaterials in the body of a patient without any cytotoxicity. It an objective to provide functionalized bone implant materials, in particular functionalized ceramic foams and porous materials, which allow for vascularization across pores of the material and/or which allow seeding with fetal cells. This approach would allow the colonization of the entire implant especially the inner part, which is often critical for the treatment of large bone defects. The covalent linkage of cells with the biomaterial may avoid cell migration to the surface.

The present invention addresses the problems depicted above.

### Summary of Invention

In an aspect, the invention provides a compound of formula (I) below: 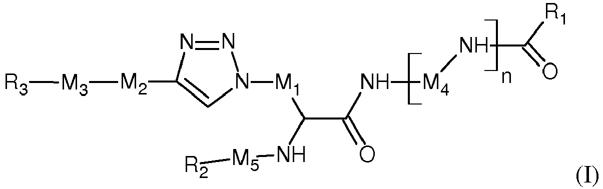 wherein M₁, M₂, M₃, M₄, M₅, R₁, R₂, R₃ and n are as defined elsewhere in this specification.

In an aspect, the invention provides a compound of formula (I) above, wherein:
M₁, M₂ and M₄ are, independently, selected from organic moieties consisting of 1 to 21 carbon atoms and from 0 to 15 heteroatoms;
R₃ is selected from a substituent comprising a peptide, saccharide, peptidoglycan, glycopeptides, and/or analogues of any one of the aforementioned;
M₃ is selected from:
   - NH-CO- if R₃ comprises a peptide, peptidoglycan or glycopeptide substituent, bound by way of amide bond M₃ formed with a free amino group of said peptide, peptidoglycan or glycopeptide, respectively; and
   - O-, if R₃ comprises a saccharide, a peptidoglycan or a glycopeptide substituent, bound by way of a ether bond formed with a primary hydroxyl group of a monosaccharide moiety of said saccharide, peptidoglycan or glycopeptide, respectively;
n is 0 or an integer from 1 to 3;
R₁ is an organic substituent comprising from 1 to 20 carbon atoms and 1 to 10 heteroatoms, said organic substituent comprising at least one anchoring group;
M₅ is selected from moieties of formulae (1) and (2) below: 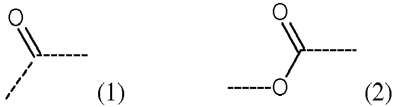
wherein
   in the moieties of formulae (1) and (2) the dotted line on the right represents the bond by which M₅ is bound to the -NH- moiety in the structure of formula (I) and the dotted line on the left represents the bond to R₂;
R₂ is selected from any one of substituents (i) to (iv) below:
   i. organic substituents comprising from 3 to 35 carbons and from 0 to 15 heteroatoms and comprising at least one carbon-carbon triple bond;
   ii. a substituent of formula (3) below: 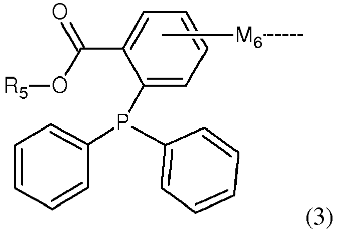 wherein the dotted line represents the bond by which R₂ is connected to the -CO- or -O-CO- group of the moiety of formulae (1) or (2), respectively;
      M₆ is an organic moiety consisting of 1 to 30 carbon atoms and 0 to 20 heteroatoms; and, R₅ is an organic substituent comprising from 1 to 20 carbons and from 0 to 10 heteroatoms;
   iii. a substituent of formula (4) below: 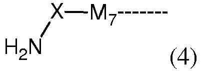 wherein -X- is selected from -NH- and from -O-, and M₇ is an organic moiety comprising from 1 to 10 carbon atoms and 0 to 5 heteroatoms; and,
   iv. a substituent of formula (5) below: 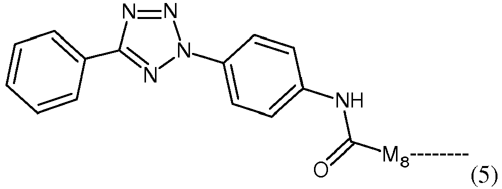
   wherein M₈ is an organic moiety comprising from 1 to 10 carbon atoms and from 0 to 5 heteroatoms, and any one or more of the hydrogens of the phenyl moiety and/or the phenyl substituent in the structure of formula (5) may be substituted by halogen or C1-C8 linear, branched and/or cyclic alkyl.

In another aspect, the invention provides a biomaterial and/or implant material comprising a compound of the invention.

In another aspect, the invention provides the use of a compound of formula (XX), or of a derivative thereof, in the synthesis of an organic linker, 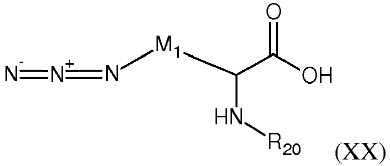 wherein R₂₀ is H or a protective group, in particular an amine protecting group; and M₁ is an organic moieties comprising from 1 to 21 carbon atoms and from 0 to 15 heteroatoms. The N-=N⁺=N- group is also referred to as a N₃- group in this specification.

In an aspect, the invention provides a method of preparing an organic linker, the method comprising the steps of providing a compound of formula (XX), or of a derivative thereof, and reacting said compound, or said derivative thereof, with at least one compound selected from the group consisting of:
- a compound comprising an anchoring group;
- a compound comprising one selected from a saccharide, a peptide, a peptidoglycan, a glycopeptide, and an analogue of any one of the aforementioned; and
a compound selected from the group consisting of: a compound having from 3 to 35 carbons and from 0 to 15 heteroatoms and comprising at least one carbon-carbon triple bond; an ortho substituated triphenylphosphine, a hydrazine, a hydrazide, an alkoxyamine and a biaryltetrazole, wherein said ortho substituated triphenylphosphine, hydrazine, hydrazide, alkoxyamine and biaryltetrazole may be further substituted.

Remarkably, the present invention provides compounds that are able to promote the adhesion and proliferation of both bone cell precursors and vascular cell precursors, and that can be conveniently conjugated to biomaterials so as to obtain functionalized implant materials, for example. The compounds can be synthesized conveniently and in high yields from commercially available starting materials. The implant materials of the invention are particularly suitable as bone implants.

Further aspects and preferred embodiments of the invention are defined herein below and in the appended claims. Further features and advantages of the invention will become apparent to the skilled person from the description of the preferred embodiments given below. The problems, objectives and goals underlying the invention discussed in the previous chapter are also part of the present invention.

### Brief Description of the Drawings

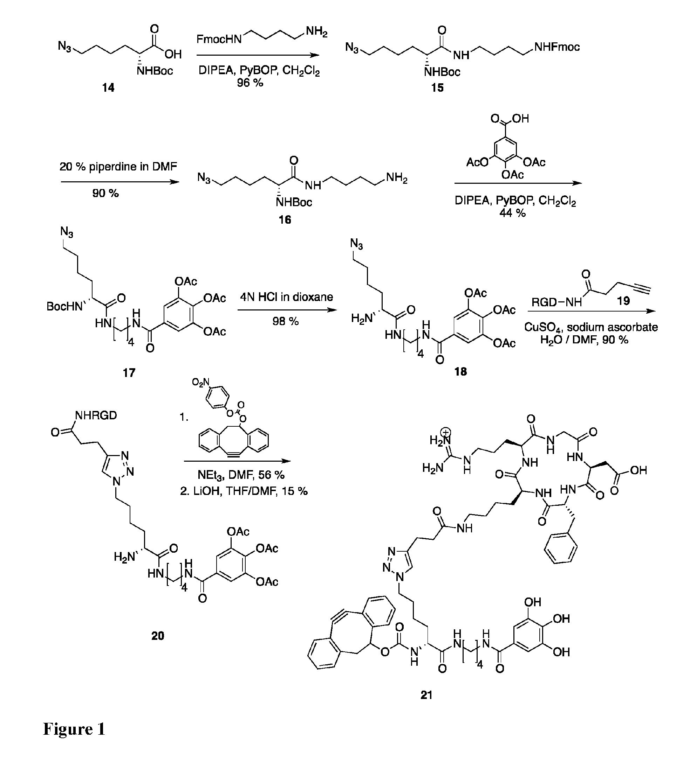

**Figure 1** shows the reaction scheme for the synthesis of an exemplary linker compound (21) as described in Example 1 in accordance with an embodiment of the invention.

### Detailed Description of the Preferred Embodiments

The compound of the invention comprises at least three substituents R₁, R₂, R₃, which fulfill different functions and/or purposes.

For the purpose of the present specification, the expression "comprising" and various grammatical forms thereof is intended to mean "includes, amongst other". It is not intended to mean "consists only of".

In principle, it is not relevant, which of the three substituents R_{1,} R₂, R₃, fulfills which functionality. It is preferred that all three different functionalities are present and/or accomplished in the compound of the invention.

A first substituent selected from said substituents R₁, R₂ or R₃ preferably functions as an anchoring group. A second substituent selected from of said substituents R_{1,} R₂ or R₃ preferably functions as a group allowing for binding to a particular type of cells, for example a first type of cells, for example by covalent binding to cells that were modified or adapted to be able to bind to the substituent. A third of said substituents R₁, R₂ or R₃ preferably functions for binding to another, for example a second type of cells, for example by noncovalent, affinity binding. Said third of said substituents preferably comprises a peptide or a saccharide having an affinity with a receptor or another cell-surface molecule. For example, any one of R₁, R₂ or R₃ may be an anchoring group, as long as any other one of said three substituents comprises a group allowing for binding to said first type of cells and the remaining substituent allows for binding to said second type of cells.

The anchoring group is preferably adapted for binding to or capable of being bound to the surface of a biomaterial. Said first type of cells is preferably selected from bone cells, osteogenic cells, or progenitors of the aforementioned, such as fetal osteoblasts or other types of osteoblast precursor cells. Said second or other type of cells is preferably selected from endothelial cells or progenitors of endothelial, in particular vascular cells.

According to an embodiment, the compound of the invention comprises at least three substituents R₁, R₂, R₃, wherein one of said three substituents, for example a first substituent, comprises an anchoring group. One other of said three substituents, for example a second substituent, comprises structure, group and/or functionality selected from the group consisting of: a structure having from 3 to 35 carbons and from 0 to 15 heteroatoms and comprising at least one carbon-carbon triple bond, an ortho substituated triphenylphosphine, a hydrazine, a hydrazide, an alkoxyamine and a biaryltetrazole. One other of said three substituents, for example a third substituent, comprises one selected from a saccharide, a peptide, a peptidoglycan, a glycopeptide, and an analogue of any one of the three aforementioned. Preferably, said substituent comprising said second substituent allows for covalent binding to said osteogenic cells or progenitor cells thereof, and said third substituent is capable of binding to endothelial cells or progenitors thereof, as specified in more detail elsewhere in this specification.

Herein below, R₁ will be taken as an exemplary substituent containing an anchoring group, R₂ will be taken as an exemplary substituent capable of binding, preferably covalently, to a first type of cells, and R₃ will be taken as an exemplary substituent capable of binding, preferably non-covalently, to a second type of cells. Specific, exemplary structures that fulfill these functions and further exemplary functional definitions of these substituents will be given below and elsewhere in this specification, while it has to be kept in mind that the positions of R₁, R₂ or R₃ in the compound of formula (I), (II), (IIa), (III), and (IIIa), for example, may be exchanged in accordance with the above.

In the compound of formula (I) and other formulae disclosed in this specification, M₁, M₂ and M₄ are, independently, selected from organic moieties consisting of 1 to 21 carbon atoms and 0 to 15 heteroatoms, preferably 1 to 17 carbons and 0 to 8 heteroatoms, and most preferably 1 to 5 carbons and 0 to 3 heteroatoms.

A "moiety", for the purpose of the present specification, is a multivalent, in particular a bivalent radical or moiety. It is generally bound with two separate bonds, preferably two separate single bonds, to two neighboring radicals, moieties and/or substituents. Generally, for the purpose of this specification, a "substituent" is a monovalent radical or substituent, generally bound by way of one single or, possibly, double bound to a neighboring structure or substituent, unless otherwise indicated.

One or more "heteroatoms" may be present, for example in moieties or substituents of the compounds of the invention. For the purpose of the present specification, a heteroatom is any atom other than H and C. Metals are also heteroatoms. According to a preferred embodiment of the invention, a heteroatom is any atom selected from any one of groups 13 to 17 of the periodic table with the exception of H and C. Preferably, the heteroatoms are selected from halogen (F, Cl, Br, I), B, N, P, O, S, Se, more preferably from F, Cl, Br, N, P, O, and S and most preferably from F, Cl, N, and O. In an organic moiety or substituent of the compounds and structures of the invention, the heteroatom may be present in the form of a substituent, for example basically substituting a hydrogen atom such as in the case of halogen, -OH, -NH₂, or may replace one or more carbon atoms in a chain of atoms, for example R-O-R, R-O-NH-R, R-O-CO-R, R-O-CO-NH-R, R-NRR, R-O-CO-R R-S-R, R-S-S-R, R-CO-NH-R and the like.

According to an embodiment, the moieties M₁, M₂ and M₄ are, independently, selected from aliphatic and/or aromatic hydrocarbon moieties consisting of 1 to 15 carbon atoms, preferably 1 to 10 and most preferably 1 to 6 carbon atoms.

According to an embodiment, M₁, M₂ and M₄ are, independently, selected from (-CH₂)ᵣ-, (-CH₂-CH₂-O)ᵣ-CH₂- and -CH₂(-O-CH₂-CH₂)ᵣ-, and M₁ and M₂ are further selected from (-CH₂-CH₂-O)ᵣ, wherein r is, independently for M₁, M₂ and M₄, an integer of 1 to 10. Preferably, r is an integer from 1 to 8, preferably from 1 to 6, most preferably from 1 to 4. According to a preferred embodiment, M₁, M₂ and M₄ are (-CH₂)ᵣ-, with r being, independently for M₁, M₂ and M₄, an integer of 1 to 5.

According to an embodiment, the moiety M₃ is preferably selected in dependence of the substituent R₃. The moiety M₃ preferably represents the bond by which M₂ is connected to substituent R₃.

According to a preferred embodiment, R₃ is a substituent that favors the interaction or binding of the compound of the invention to cells of interest. In this way, the compound of the invention, when bound to a biomaterial by way of its anchoring group, favors colonization of the biomaterial, in particular on its surface, by cells of interest. Preferably, substituent R₃ favors or allows binding or interaction to endothelial cells and/or endothelial precursor cells. Preferably, substituent R₃ favors or supports the colonization of a biomaterial containing the compound of the invention by endothelial cells or progenitors thereof, more preferably by vascular cells or progenitors thereof. Preferably, substituent R₃ favors or promotes the vascularization of the biomaterial functionalized on the surface by the compound of the invention. For example, R₃ promotes colonization of the biomaterial by Human umbilical vein endothelial cells (HUVECs).

Preferably, R₃ comprises a substituent that is recognized and/or that binds with molecules on the surface of endothelial cells or progenitors thereof, in particular vascular cells or progenitors thereof. The binding is preferably not covalent and/or is based on affinity binding between R₃ and molecules, such as receptors, saccharides, and so forth, present on the surface of said endothelial cells.

R₃ is preferably selected from substituents comprising peptides, peptidoglycans, glycopeptides, saccharides, preferably oligo- and/or polysaccharides, and analogues of the aforementioned. According to an embodiment, said analogues are preferably analogues of the peptide part of said peptides, peptidoglycans and glycopeptides. For example, R₃ may be selected from saccharides, peptides, peptide analogues, peptidoglycans, peptide analogues of peptidoglycans, glycopeptides and peptide analogues of glycopeptides. According to another embodiment, said analogues are analogues of the saccharide part of said saccharides, peptidoglycans and glycopeptides.

If R₃ is or comprises a peptide, peptidoglycan, glycopeptides or an analogue thereof, it may be bound by way of a peptide bond with or to M₂. In this case, M₃ preferably is -NH-CO-, wherein the nitrogen in said peptide bond stems from a free amino group of an amino acid in said peptide, peptidoglycan, glycopeptides or an analogue thereof. If R₃ is a peptidoglycan, glycopeptide or saccharide, it may be bound by way of an ether bond with or to M₂. In this case, M₃ preferably is -O-, formed with a primary hydroxyl group of a monosaccharide moiety or substituent of said peptidoglycan, glycopeptide or saccharide.

According to an embodiment, R₃ comprises a peptide substituent selected from the group consisting of: an RGD peptide, a cadherin, a catenin, an integrin, collagen IV, an elastin, a fibronectin, a laminin and a peptide sequence of at least three continuous amino acid moieties taken from the amino acid sequence of any one of the aforementioned peptides and/or proteins, and analogues of any one of the aforementioned.

According to an embodiment, R₃ comprises a peptide substituent selected from the group consisting of: (a) an RGD peptide or an analogue thereof, (b) a peptide comprising an amino acid sequence of at least three, preferably at least 4, 5, 6, 7, 8, 9, or at least 10 continuous amino acid moieties of an amino acid sequence of a peptide selected from the group consisting of: a cadherin, integrin, collagen IV, an elastin, a fibronectin, and a laminin, and (c) an amino acid sequence having at least 60% sequence identity with the amino acid sequence defined in (b). Preferably, said sequence identity is at least 70%, more preferably at least 80%, even more preferably at least 90% and most preferably at least 95%.

For the purpose of the present specification, sequence identity percentage is determined by using the basic protein blast on the internet (http://blast.ncbi.nlm.nih.gov) with preset standard parameters and database selections. This sequence comparison tool is based on algorithms detailed in the following publications: Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402. Stephen F. Altschul, John C. Wootton, E. Michael Gertz, Richa Agarwala, Aleksandr Morgulis, Alejandro A. Schäffer, and Yi-Kuo Yu (2005) "Protein database searches using compositionally adjusted substitution matrices", FEBS J. 272:5101-5109.

Standard parameters include the selection of blastp (protein-protein BLAST, automatic adjustment of parameters to short input sequences; expect threshold 10, word size 3, use of the matrix BLOSUM62; Gap costs: existence: 11, extension 1; conditional compositional score matrix adjustment, no filters and no masking). For short sequences parameters may be adjusted.

Sequence identity of a sequence of comparison with respect to an original sequence is reduced when, for example, any one of the compared or the original sequence lacks amino acid residues, has additional amino acid residues and/or has one or more amino acid residue substituted by another residue. Sequences having as little as 50% sequence identity with any sequence as defined herein may still be functional.

According to an embodiment, if R₃ comprises a peptide, it comprises preferably a stretch of from 2 to 200, preferably from 3 to 100, even more preferably from 3 to 50 and most preferably from 3 to 20 amino acid moieties forming said peptide. Said peptide may thus be an oligopeptide, such as a dipeptide, a tripeptide, etc. or a peptide comprising up to 10 amino acids, or a polypeptide, containing a stretch of more than 10 amino acids.

According to an embodiment, R₃ comprises from 8 to 400 carbon atoms and from 8 to 300 heteroatoms, preferably from 8 to 300 carbon atoms and from 8 to 200 heteroatoms, more preferably from 8 to 200 carbon atoms and from 8 to 150 heteroatoms, even more preferably from 10 to 100 carbons and from 10 to 50 heteroatoms, and most preferably from 10 to 50 carbons and from 10 to 40 heteroatoms.

According to an embodiment, R₃ comprises an RGD peptide or analogue. RGD peptides are peptides comprising at least the tripeptide amino acid sequence Arg-Gly-Asp, as such or embedded in a larger peptide, for example in an oligo- or polypeptide. RGD peptides may be cyclic or linear, for example. Many different forms of RGD peptides are commercially available.

According to an embodiment, M₃ is -NH-CO-, and R₃ is a cyclic RGD-peptide substituent of formula (30) below: 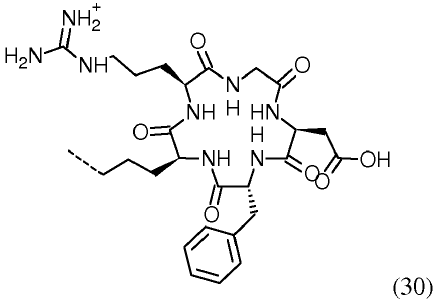 wherein the dotted line in formula (30) represents the single bond by which R₃ is connected to the -NH- moiety of M₃. This cyclic RGD peptide comprises, besides the RGD sequence, a D-Phe and a Lysine moiety, resulting in a cyclic(Arg-Gly-Asp-D-Phe-Lys) peptide. The cyclic RGD peptide is preferably bound by the free amino group at the ε carbon in the side chain of the lysine moiety in the compound of the invention, forming said substituent as R₃, wherein the nitrogen atom of this amino group, not shown in substituent (30), is the nitrogen in the peptide bond of M₃.

According to an embodiment, R₃ comprises (a) an amino acid sequence stretch of at least 8, preferably at least 10, most preferably at least 15 continuous amino acids taken from a collagen IV or (b) a an amino acid sequence having at least 70%, 80%, preferably at least 90% and most preferably at least 95% sequence identity with said amino acid stretch (a).

Preferably, said collagen IV is a proteins encoded by any one of the human genes COL4A1, COL4A2, COL4A3, COL4A4, COL4A5, and COL4A6, but said collagen IV may also be a collagen IV encoded by an animal gene.

According to an embodiment, R₃ comprises at least the amino acid sequence (SEQ ID NO: 1) GVKGDKGNPGWPGAP of collagen IV, or an amino acid sequence having at least 70%, 80%, preferably at least 90% and most preferably at least 95% sequence identity with SEQ ID NO: 1.

According to an embodiment, R₃ comprises (a) an amino acid sequence stretch of at least 3, preferably at least 4, most preferably at least 5 continuous amino acids taken from a laminin or (b) a an amino acid sequence having at least 70%, 8%, preferably at least 90% and most preferably at least 95% sequence identity with said amino acid stretch (a).

According to an embodiment, R₃ comprises at least the amino acid sequence YIGSR (SEQ ID NO: 2) or IKVAV (SEQ ID NO: 3) of laminin.

According to an embodiment, R₃ comprises (a) an amino acid sequence stretch of at least 5, preferably at least 10, most preferably at least 15 continuous amino acids taken from a cadherin or a catenin, or (b) a an amino acid sequence having at least 70%, 80%, preferably at least 90% and most preferably at least 95% sequence identity with said amino acid stretch (a). Said cadherin or catenin peptide is preferably selected from an α-catenin, β-catenin and VE-cadherin.

If R₃ is a saccharide, it may be selected from hyaluronic acid, heparan sulfate, and chondroitin sulfate, for example.

R₃ may also be selected from proteoglycans comprising saccharide chains, such as peptides bound to heparan sulfate, and chondroitin sulfate, glycosaminoglycan chains in general, for example.

R₁ is preferably an organic substituent comprising from 1 to 20 carbon atoms and 1 to 10 heteroatoms, said organic substituent comprising at least one anchoring group. The anchoring group is suitable to anchor or bind said compound on the surface of a biomaterial, for example.

According to an embodiment, any anchoring group is selected from -OH, -COOH, -PO₃H₂, - PO₄H₂, -P(R₄)O₂H (phosphinic acid); -SO₃H₂, -SO₄H₂, -CONHOH⁻, 1,2 hydroxy benzene, 1-hydroxy-2-carboxy benzene, acetylacetonate, deprotonated forms of the aforementioned, organic and/or inorganic salts of said deprotonated forms, and chelating groups with π-conducting character. R₄ is preferably H or an organic substituent comprising from 1-20 carbons and 0-10 heteroatoms, preferably 1-10 carbons and 0 to 5 heteroatoms. Preferably, R₄ is an aliphatic or aromatic substituent comprising from 1-20, preferably 1-10 carbon atoms. According to an embodiment, R₁ comprises an aromatic moiety on which is provided one or more anchoring groups selected, independently, from -OH, -COOH, -PO₃H₂, -PO₄H₂, - P(R₄)O₂H (phosphinic acid); -SO₃H₂, -SO₄H₂, -CONHOH⁻, acetylacetonate, deprotonated forms of the aforementioned, organic and/or inorganic salts of said deprotonated forms. The aromatic moiety may comprise one or more rings, for example a combination of several fused rings forming an aromatic ring system. Preferably, the ring or ring system of said aromatic moiety is free of a ring heteroatom. Examples of preferred aromatic moieties are benzene, pentalene, indene, naphthalene, azulene, indacene, and anthracene. Preferably, said aromatic moiety is benzene.

According to a preferred embodiment, said one or more anchoring groups are two or more - OH provided on said aromatic moiety.

According to an embodiment, R₁ is a substituent of formulae (20) below: 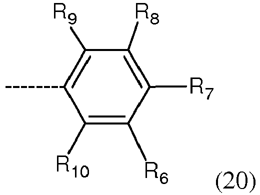 wherein:
R₆ to R₁₀ are independently selected from H, halogens, and from C1-C10 substituents comprising from 0 to 5 heteroatoms, with the proviso that at least one and, in the case of the anchoring group -OH: at least two of R₆ to R₁₀ comprises or consists of an anchoring group,
as defined above. Preferably said anchoring group is selected from -OH and -COOH, most preferably said anchoring group is -OH.

According to an embodiment, R₁ is selected from substituents of formulae (21) and (22) below:

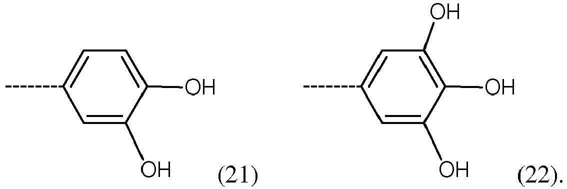

According to a preferred embodiment, R₂ is a substituent that allows the binding of the compound of the invention to cells of interest. In this way, the compound of the invention, when bound to a biomaterial by way of its anchoring group, favors colonization of the biomaterial, in particular on its surface, by cells of interest.

According to an embodiment, R₂ allows, favors or promotes binding and/or interacting to cells that promote bone tissue growth and/or bone tissue formation, and/or precursor cells of such cells. By favoring colonization of the biomaterial with such cells, the compound of the invention contributes to the integration of the biomaterial in natural bone tissue and/or favors bone repair.

According to an embodiment, R₂ allows, favors or promotes binding to bone cells, bone progenitor cells, osteogenic or osteoprogenitor cells and/or mesenchymal stem cells. According to an embodiment, R₂ allows, favors or promotes binding to osteoprogenitor cells, in particular to bone fetal osteoblasts.

According to an embodiment, said cells of interest that can bind to R₂ are treated and/or modified so as to bind to said substituent. Preferably, said cells contain modifications at their surface, so as to allow interaction and/or chemical reaction with R₂.

According to an embodiment, M₅ is selected from moieties of formulae (1) and (2) below: 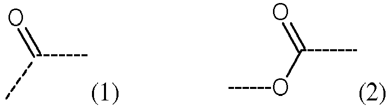 wherein, in the substituents of formulae (1) and (2) the dotted line on the right side represents the bond by which M₅ is bound to the respective -NH- moiety in the structure of formula (I). In accordance with this embodiment, R₂ is the substituent that fulfills the functionality of binding to cells of interest, as detailed elsewhere in this specification. Formulae (1) and/or (2) represent the bond or a part thereof by which R₂ is bound to the general structure of formula (I). Preferably, R₂ is bound by way of an amide (1) or carbamate (2) bond, respectively, to the general structure of formula (I).

According to an embodiment, R₂ allows for covalent binding to said cells of interest. According to an embodiment, R₂ allows for covalent binding to cells expressing azido-modified proteins, for example osteogenic cells or progenitors thereof expressing azido-modified proteins.

According to an embodiment, R₂ is selected from organic substituents comprising from 3 to 35 carbons and from 0 to 15 heteroatoms and comprising at least one carbon-carbon triple bond. In accordance with this embodiment, R₂ allows for covalent binding to cells expressing azido-modified proteins, in particular by click-reaction, for example copper-mediated or copper-free click reaction. Preferably, the carbon-carbon triple bond in R₂ is suitable to allow for copper-free click reaction with cells expressing azido-modified proteins, preferably under physiological conditions, thereby creating a covalent bond with said cells.

According to an embodiment, said substituent (R₂) comprising from 3 to 35 carbons and from 0 to 15 heteroatoms and comprising at least one carbon-carbon triple bond comprises a structure of formula (6) below: 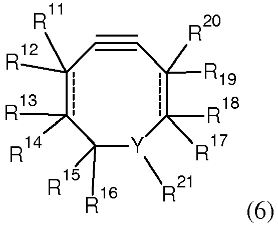 wherein:
Y is N or CR²², R²² being selected, independently, as defined for substituents R¹¹ to R²¹, with the proviso that if Y is N, R²¹ is selected from H and substituents -CH₂-R²²;
R¹¹ to R²¹ are independently selected from H, halogen, -OH, -NH₂, and organic substituents comprising from 1 to 20 carbon atoms and 0 to 15 heteroatoms, wherein, a substituent pair of substituents on the same ring carbon (for example, R¹¹ and R¹²) can form any one selected from: an =O (oxo group), a carbon-carbon double bond (such as =CR^{11'}R^{12'}, in case of R¹¹ and R¹², for example), and a substituent comprising a delocalized π-bond; wherein R^{11'} and R^{12'}, and so forth, as applicable, are independently selected from H, halogen, and from organic substituents comprising from 1 to 19 carbon atoms and 0 to 15 heteroatoms, wherein substituents on neighbouring ring carbons, including a substituent =CR^{11'}R^{12'} and so forth, as applicable, with one or two substituents on a neighbouring ring carbon, may form a ring fused to the 8-atom ring in formula (6);
wherein said combined dotted and solid lines in the 8-atom ring each represent, independently, a single bond, a single bond and a delocalized π-bond, or a double bond, wherein, if any one or both of said dotted and solid lines are double bonds, the substituents R¹² and R¹⁴, and/or R¹⁸ and R²⁰, respectively, are absent, so as to observe to an applicable valence, and wherein, if any one or both of said dotted and solid lines contain delocalized π-bonds, at least one of the substituent pairs R¹¹/R¹² and R¹³/R¹⁴, and/or, respectively, R¹⁷/R¹⁸ and R^{19/}R^{20,} also contain delocalized π-bonds and substituent pairs on neighbouring ring carbons may be connected with each other to form an aromatic system fused to the 8-atom ring of formula (6); and,
wherein at least one of said substituents R¹¹ to R²¹ and R²² is a either moiety of formula (7) below:
   -------M₉------- (7), or directly connects the structure of formula (6) by way of single bond of a ring carbon of the 8-atom ring or of a -CH₂- moiety in said structure (in case of -CH₂-R²²), to the carbon or oxygen, respectively, in the moieties of formulae (1) and (2);
   wherein M₉ is an organic moiety comprising from 1 to 10 carbon atoms and from 0 to 5 heteroatoms, and the dotted lines connected on the left and right sides to M₉ represent the bonds by which M₉ is connected to the structure of formula (6) and to the moiety of formula (1) or (2).

In accordance with the above, substituent pairs on a given ring carbon may be provided in the form of =CR^{11'}R^{12'}, in case of R¹¹ and R¹²; =CR^{13'}R^{14'}, in case of R¹³ and R¹⁴; =CR^{15'}R^{16'} in case of R¹⁵ and R¹⁶; =CR^{17'}R^{18'}, in case of R¹⁷ and R¹⁸; and =CR^{19'}R^{20'}, in case of R¹⁹ and R²⁰, wherein R^{13'} to R^{20'} are defined, independently, as R^{11'} and R^{12'}.

Preferably, in the substituent of formula (6), substituents on ring carbons next to the triple bond (R¹¹/R¹² and R¹⁹/R²⁰) do not carry an -OH or -NH₂ group, whereas R¹³⁻¹⁸, preferably R¹⁵⁻¹⁶ may be selected from -OH, -NH₂, besides the organic substituents comprising from 1 to 20 carbon atoms and 0 to 15 heteroatoms, as specified above.

Preferably, the substituents on ring carbons next to the triple bond (R¹¹/R¹² and R¹⁹/R²⁰) contain or consist of an electron withdrawing group and/or contain a double bond (C=O; =CR^{11'}R^{12'} or a bond containing a delocalized π-bond, so as to increase the reactivity and/or the ring strain in the 8-membered ring of formula (6). In this way, the reactivity of the triple bond is increased, allowing for binding to cells by click reaction as described below, preferably in the absence of a (copper) catalyst.

An electron withdrawing group or function is a group that has an electron pulling character, a region of low electron density and/or an electron poor group, so that the group is capable of taking an electron up, thereby becoming less electron poor, reducing the electron density. Preferred examples of electron acceptors are halogen, -CN, and substituents that are in a π-conjugated connection with the structure of formula (6), in particular with the triple bond in said structure. If any one or more of R¹¹/R¹² and R¹⁹/R²⁰ contain an electron withdrawing group, the reactivity of the triple bond is increased.

When reacting with cells expressing azido-modified proteins by click-reaction, the cells are preferably bound to said substituent of formula (6) as shown in scheme (6a) below:

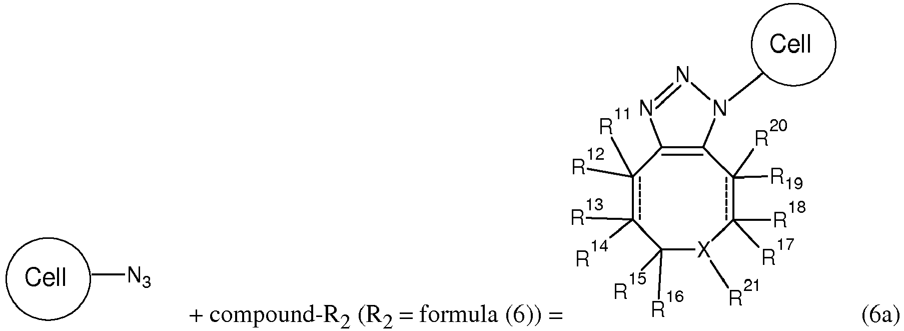

According to an embodiment, said substituent R₂ comprising from 3 to 35 carbons and from 0 to 15 heteroatoms and comprising at least one carbon-carbon triple bond comprises and/or consists of any one selected from the structures of formulae (8) to (12) below: 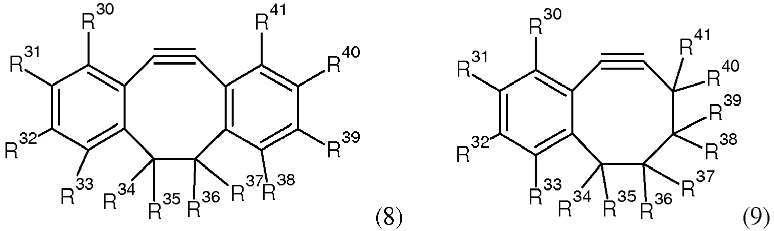 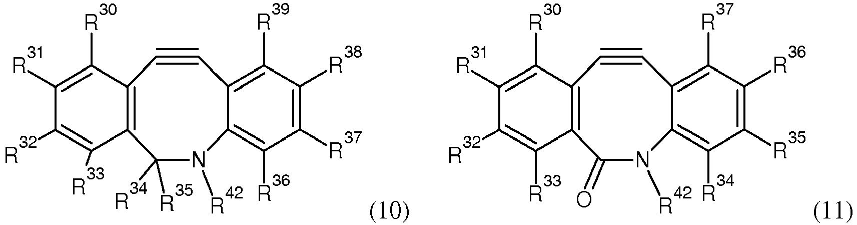 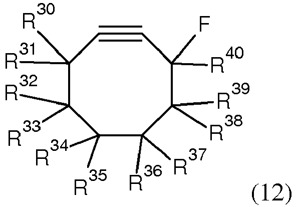 wherein R³⁰ to R⁴¹, in as far as present, are independently selected from H, halogen, -OH, - NH₂, and organic substituents comprising from 1 to 10 carbon atoms and 0 to 5 heteroatoms and wherein a pair of substituents on the same ring carbon (for example R³⁰ and R³¹ in the structure of formula (12)) may form an oxo group (=O); wherein R⁴² is selected from H and substituents -CH₂-R⁴³, R⁴³ being selected, independently, as defined for substituents R³⁰⁻⁴¹; and, wherein at least one of said substituents R³⁰⁻⁴¹; R³⁰⁻⁴¹; R³⁰⁻³⁹ and R⁴³; R³⁰⁻³⁷ and R⁴³; and R³⁰⁻⁴⁰ in formulae (8); (9); (10); (11); and (12), respectively, is either a linker moiety of formula (7) below:

------M₉------ (7)

, or ,
directly connects the structure of formula (8), (9), (10), (11) or (12), by way of single bond of a ring carbon or of a -CH₂- moiety in said structures (in case of -CH₂-R⁴³), to the carbon or oxygen atom, respectively, in the moieties of formulae (1) and (2);
wherein, in the moiety of formula (7), M₉ is an organic moiety comprising from 1 to 15 carbon atoms and from 0 to 10 heteroatoms, and the dotted lines connected on the left and right sides to M₉ represent the bonds by which M₉ is connected to one of the structures of any one of formulae (8) to (12) and, on the other hand, to the moiety of formula (1) or (2), respectively.

According to an embodiment of the substituent of any one of formulae (6) and (8) to (12), in said moiety of formula (7), M₉ is absent or an organic moiety comprising from 1 to 11 carbon atoms and from 0 to 5 heteroatoms, more preferably 1 to 5 carbons. According to an embodiment, M₉ is absent or an aliphatic moiety of 1-15, preferably 1-11, and most preferably 1- 5 carbon atoms.

According to an embodiment, M₉ is selected from (-CH₂)ₛ-, (-CH₂-CH₂-O)ₛ-CH₂- and - CH₂(-O-CH₂-CH₂)ₛ-,wherein s is 0 or an integer of 1 to 10, preferably 1 to 5 and most preferably 1 to 3.

According to an embodiment, said substituent R₂ comprises and/or consists of any one selected from the structures of formulae (13) to (18) below: 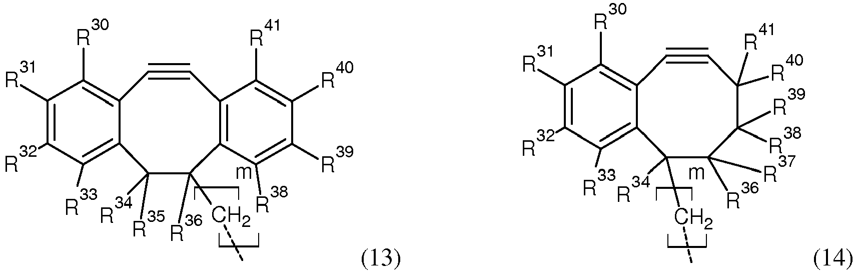 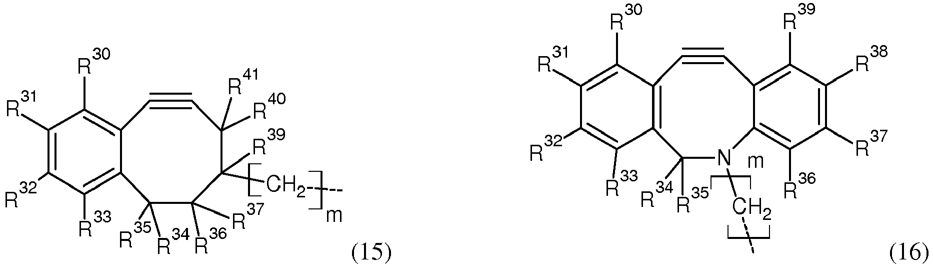 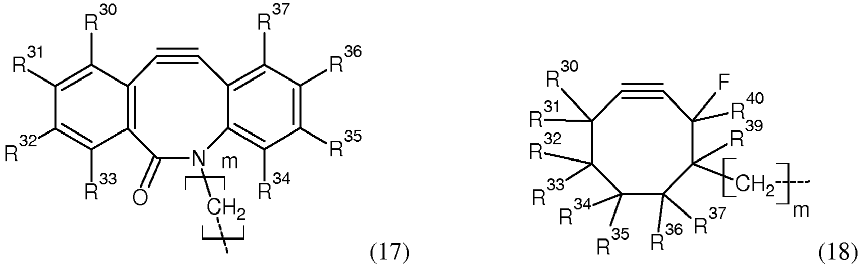 wherein
m is an integer of 1 to 5 and, in formulae (13), (14), (15), and (18) may also be 0 (zero), and R³⁰ to R⁴¹, in as far as present, are independently selected from H, halogen, -OH, -NH₂, and organic substituents comprising from 1 to 10 carbon atoms and 0 to 5 heteroatoms, wherein a pair of substituents on the same ring carbon (for example R³⁰ and R³¹ in the structure of formula (18) or R³⁴ and R³⁵ in the structures of formulae (13), (15), (16), and (18)) may form an oxo group (=O), and wherein the dotted line represents the bond or radical by which the structure of any one of formulae (13) to (18) is connected to the moiety of formula (1) or (2). Preferably, m is 0 (not in (16) or (17)) or an integer of 1 to 3, most preferably m is 0 or 1.

As mentioned above with respect to the structure of formula (6), in the structures of formulae (9), (14), (15), and (18) substituents on carbons next to the triple bond are preferably different from -OH, and -NH₂. Preferably, these substituents comprise and/or consist of an electron withdrawing group, other substituents suitable to increase the reactivity of the triple bond, or H or other substituents as defined but different from -OH and -NH₂.

According to an embodiment, R³⁰ to R⁴³ in any one of said substituents of formulae (8) to (12) and (13) to (18), in as far as present, are independently selected from H, halogen, hydroxyl, carboxyl, -CN, C1-C10 alkyl, C2-C10 alkenyl, C2-C10 alkynyl, C6-C10 aryl, C4-C10 heteroaryl, C1-C10 alkoxyl, C1-C10 alkoxenyl, C6-C10 aroxyl, wherein said alkyl, alkenyl, alkynyl, aryl, heteroaryl, alkoxyl, alkoxenyl, and aroxyl may or may not be further substituted, and wherein said alkyl, alkenyl, alkynyl may be linear, branched and/or cyclic. Further substituents may be selected, for example, from halogen, oxo, hydroxyl, carboxyl, - CN, C1-C5 alkyl, C1-C5 alkoxyl, C2-C5 alkenyl, C2-C5 alkenoxyl, and C2-C5 alkynyl.

According to a preferred embodiment, R³⁰ to R⁴³ in any one of said substituents of formulae (8) to (12) and (13) to (18), in as far as present, are independently selected from H, halogen, hydroxyl, carboxyl, -CN, C1-C5 alkyl, C2-C5 alkenyl, C2-C5 alkynyl, C6-C8 aryl, C4-C8 heteroaryl, C1-C5 alkoxyl, C2-C5 alkoxenyl, C6-C8 aroxyl, wherein said alkyl, alkenyl, alkynyl, aryl, heteroaryl, alkoxyl, alkoxenyl, and aroxyl may or may not be further substituted, and wherein said alkyl, alkenyl, alkynyl may be linear, branched and/or cyclic. Further substituents may be selected from halogen, oxo, hydroxyl, carboxyl, -CN, C1-C3 alkyl, C1-C3 alkoxyl, C2-C3 alkenyl, C2-C3 alkenoxyl, and C2-C3 alkynyl.

According to a still preferred embodiment, R³⁰ to R⁴³ in any one of said substituents of formulae (8) to (12) and (13) to (18), in as far as present, are independently selected from H, halogen, hydroxyl, carboxyl, -CN, C1-C5 alkyl and C1-C5 alkoxyl, wherein said alkyl and alkoxyl may be linear, branched or cyclic.

Most preferably, there are no further substituents (besides H) on the fused phenyl rings in the structures of formulae (13), (14), (16) and (17).

Most preferably, in the structures of formulae (14), (15) and (18), R⁴⁰ and R⁴¹ are halogen, preferably F.

According to another embodiment, R₂ is a substituent allowing for binding to cells expressing azido-modified proteins by way of Staudinger ligation. In particular, R₂ is a substituent comprising a triaryl phosphine on which an electrophilic trap is provided. In this case, the triaryl phosphine can bind by way of a Staudinger ligation to the azido group expressed on the surface of a cell, forming an amide bond. Preferably, said triaryl phosphine is an ortho substituated triphenylphosphine comprising an electrophilic trap.

In accordance with an embodiment, R₂ is a substituent of formula (3) below: 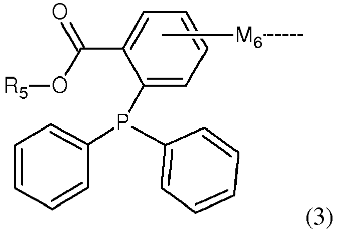 wherein the dotted line represents the bond by which R₂ is connected to the -CO- or -O-CO- group of the moiety of formulae (1) or (2), respectively;
M₆ is an organic moiety consisting of 1 to 30 carbon atoms and 0 to 20 heteroatoms, and R₅ is or an organic substituent comprising from 1 to 20 carbons and from 0 to 10 heteroatoms, preferably an organic substituent comprising from 1 to 10 carbons and from 0 to 5 heteroatoms. Preferably, R₅ is an aliphatic substituent. Preferably, R₅ is a linear, branched and or cyclic C1-C10 alkyl or C2-C10 alkenyl, more preferably a C1-C5 alkyl or C2-C5 alkenyl, which may be linear, branched and/or cyclic.

When reacting with cells expressing azido-modified proteins by Staudinger ligation, the cells are bound to said substituent of formula (3) as shown in scheme (3a) below:

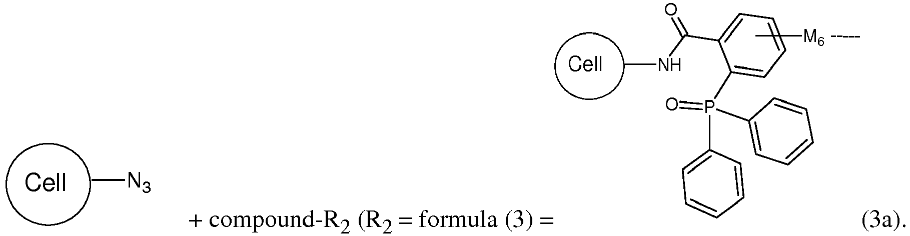

According to an embodiment, M₆ is an organic moiety consisting of from 1 to 20 carbon atoms and from 0 to 15 heteroatoms, more preferably consisting of 1 to 10 carbon atoms and from 0 to 5 heteroatoms.

According to an embodiment, selected from (-CH₂)ₜ-, (-CH₂-CH₂-O)ₜ-CH₂-, -CH₂(-O-CH₂-CH₂)ₜ-, and (-O-CH₂-CH₂)ₜ-, wherein t is an integer of 1 to 10. Preferably, t is an integer from 1 to 8, preferably from 1 to 6, most preferably from 1 to 5. According to a preferred embodiment, M₆ is (-CH₂)ₜ-, with t being an integer of 1 to 4.

According to another embodiment, R₂ is a substituent allowing for binding to cells that are modified with N-levulinoyl mannosamine, a sugar that can be inserted on the cell surface, instead of the azide as shown above. Such N-levulinoyl mannosamine-modified cells can react with a hydrazide, a hydrazine and/or a alkoxyamine group present according to an embodiment of the compound of the invention.

According to an embodiment, R₂ is a substituent of formula (4) below: 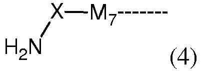 wherein -X- is selected from -NH- and from -O-, and M₇ is an organic moiety consisting of 1 to 20 carbon atoms and 0 to 15 heteroatoms, preferably 1 to 11 carbons and 0 to 7 heteroatoms, most preferably 1 to 7 carbons and 0 to 4 heteroatoms. According to preferred embodiment, if the substituent of formula (4) M₇ is a hydrazine and/or an alkoxyamine, M₇ is preferably selected from (-CH₂)ᵤ-, (-CH₂-CH₂-O)ᵤ-CH₂- and -CH₂(-O-CH₂CH₂)ᵤ-, u being an integer from 1 to 8, preferably from 1 to 5, most preferably from 1 to 3.

In case substituent (4) is a hydrazide, -X- is -NH- and M₇ starts with an acyl group, being - CO-M_{7'}-, wherein M_{7'}, is an organic moiety consisting of 1 to 19 carbon atoms and 0 to 14 heteroatoms. In this case, M_{7'} is preferably (-CH₂)ᵤ-, (-CH₂-CH₂-O)ᵤ-CH₂- and -CH₂(-O-CH₂-CH₂)ᵤ-, u being an integer from 1 to 7, preferably from 1 to 4, most preferably from 1 to 2.

When N-levulinoyl mannosamine-modified cells react with the compound of the invention in accordance with the embodiment above, a hydrazone or oxime derivative is formed, by which the compound of the invention is covalently bound to the cells. This is disclosed, for example, in Chem Commun (Camb). 2010 March 14; 46(10): 1589-1600. doi:10.1039/b925931g.

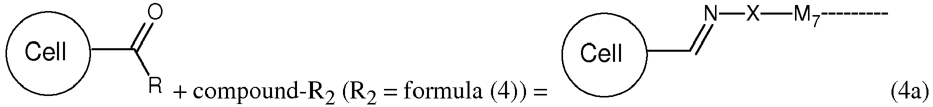

According to another embodiment, R₂ is a substituent allowing for binding to cells exhibiting a terminal alkene on their surface after modification with homoallylglycine, as disclosed, for example, in Chem Commun (Camb). 2010 March 14; 46(10): 1589-1600. doi:10.1039/b925931g. When homoallylglycine-modified cells are allowed to react wiwith the compound of the invention comprising a biaryltetrazole, a single bond is formed by a 1,3 dipolar cycloaddition, by which the compound of the invention is covalently bound to the cells.

According to an embodiment, R₂ is a substituent of formula (5) below: 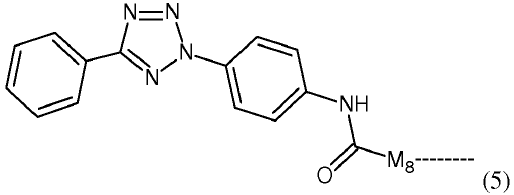 wherein M₈ is an organic moiety comprising from 1 to 10 carbon atoms and from 0 to 5 heteroatoms and any one or more of the hydrogens of the phenyl moieties and/or substituents in the structure of formula (5) may be substituted by halogen or C1-C8 linear, branched and/or cyclic alkyl, preferably C1-C4 linear, branched and/or cyclic alkyl, and most preferably said hydrogens are not substituted or are partially or totally substituted by halogen..

The dotted line on the right represents the bond by which substituent (5) is bound to moiety M₅ in formulae (I), (II), (IIa), (III), and (IIIa), for example.

When reacting with said terminal alkene on the surface of a cell of interest in presence of light, a single bond is formed, binding the cell of interest to substituent R₂ as shown in the structure of formula (5a) below. This is mentioned in in Chem Commun (Camb). 2010 March 14; 46(10): 1589-1600. doi:10.1039/b925931g.

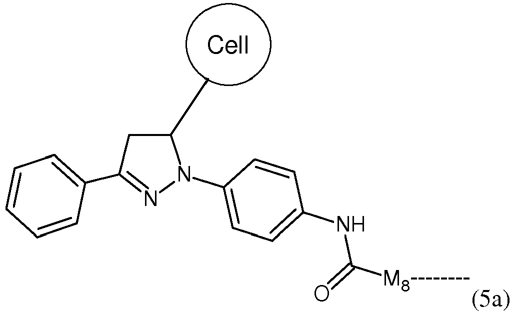

According to an embodiment, the compound of the invention is a compound of any one formulae (II), (IIa), (III) and (IIIa) below: 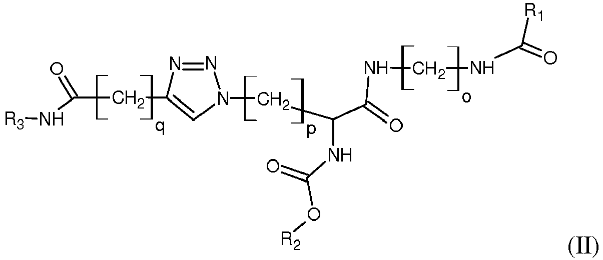 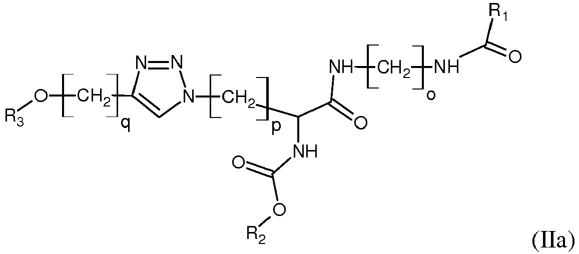 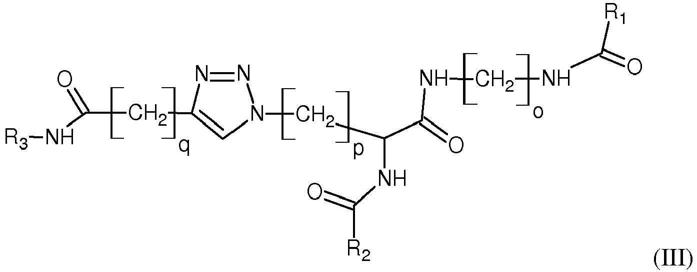 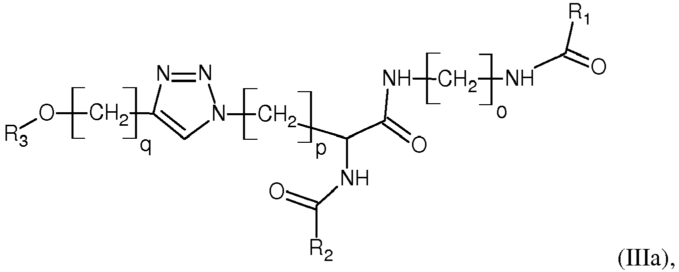 wherein o, p and q are, independently, selected from an integer of 1 to 10; and R₁, R₂ and R₃ are as defined elsewhere in this specification. Preferably, o, p and q are, independently, selected from an integer of 1-7, more preferably 1-5.

According to an embodiment, the compound of the invention is a compound of formula (IV) below:

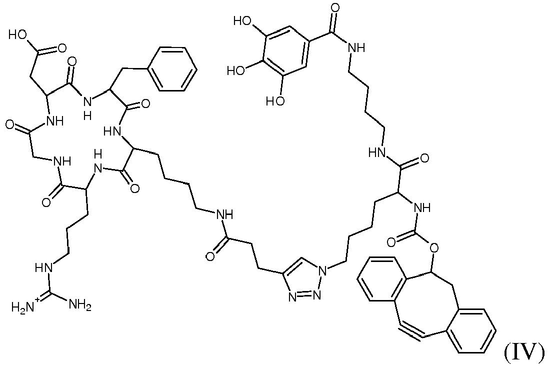

The invention encompasses a method of preparing, in particular synthetizing an organic linker, the method comprising the steps of providing a compound of formula (XX), or of a derivative thereof, and reacting said compound, or a derivative thereof, with at least one compound selected from the group consisting of: a first compound comprising an anchoring group; a second compound comprising one selected from a saccharide, a peptide, a peptidoglycan or a glycopeptide; and a third compound selected from: a compound comprising from 3 to 35 carbons and from 0 to 15 heteroatoms and comprising at least one carbon-carbon triple bond, a compound comprising an ortho substituated triphenylphosphine, a hydrazine, a hydrazide, an alkoxyamine and a biaryltetrazole. Preferably, said compound (XX) or said derivative, is reacted with all three of said compounds, preferably successively, thereby providing three different substituents at carboxy, amino and azide group of said compound (XX) or said derivative.

An example of a derivative of a compound of formula (XX) is, for example, a compound of formula (XXI) below: 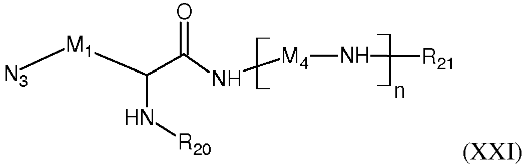 wherein M₁ and M₄ and n are as defined elsewhere in this specification, in particular with respect to the compound of formula (I) above, and, by analogy, in the compounds of formulae (II), (IIa), (III), and (IIIa); and R₂₀ and R₂₁ are selected, independently, from H and from amine protecting groups. An example of a derivative of a compound of formula (XX) is the compound 15 in the scheme shown in Fig. 1.

According to an embodiment of the method of synthetizing an organic linker, the method preferably comprises reacting the compound of formula (XX), or of a derivative thereof, with all three selected from said first, second and third compounds, one after the other in any desired or convenient order, or simultaneously, so that covalent bonds or moieties connecting the starting material of formula (XX), or the derivative thereof, and said first, second and third compounds are formed.

The present invention provides biomaterials, implant materials, prosthetics and the like. The biomaterials preferably comprise the compound of the invention. Preferably, the compound of the invention is bound and/or attached to the biomaterial, in particular to the surface of the biomaterial. The compounds of the invention are preferably used to modify the surface of said biomaterials and/or to functionalize said biomaterials. In this way, the objectives underlying the present invention are addressed.

For the purpose of the present specification, the expression "biomaterial" encompasses any material to be intended for use in a human or animal body, such as implant materials, prosthetic materials, and the like. According to a preferred embodiment, the biomaterial is an implant material, which is a material intended for implantation in the human or animal body. Preferably, the biomaterial is a bone implant material.

According to a preferred embodiment, the said biomaterial comprises or consists essentially of a polymer and/or a ceramic comprising alumina.

According to a preferred embodiment, the said implant material comprises or consists essentially of a porous polymer, ceramic foam, a porous ceramic material and/or a combination comprising two or more of the aforementioned.

The present invention will now be illustrated by way of examples. These examples do not limit the scope of this invention, which is defined by the appended claims.

### Examples

### General methods for synthetic procedures

Commercial reagents (Fluka, Aldrich, VWR, Switzerland) were used without further purification. Anhydrous solvents were obtained by filtration (PureSolv MD Series, Innovative Technology). TLCs for reaction monitoring were performed on Merck silica gel 60 F254 plates, and spots were revealed with UV light and reaction with KMnO4 or ninhydrine. IR spectra were recorded on a Perkin-Elmer-1420 spectrometer. 1H-NMR spectra were recorded on a Bruker ARX-400 spectrometer (400 MHz) using CDC13 or MeOD as solvent and calibrated using the solvent's residual signal at 7.27 or 3.31 ppm as an internal reference. 13CNMR spectra were recorded on a Bruker ARX-400 spectrometer (100.6 MHz) using CDC13 or MeOD as solvent and calibrated using the solvent's residual signal at 77.0 or 49.0 ppm as an internal reference. 19F NMR spectra: same instrument as above (376 MHz) using CFC13 as solvent and calibrated using the solvent's residual signal at 0 ppm as an internal reference.

Chemical shifts are expressed in parts per million (ppm) and coupling constants (*J*) in hertz. Mass spectra were obtained on a Nermag R-10-10C spectrometer with chemical ionization (NH3) and mode m/z (amu) [% relative base peak (100%)]. Semi-preparative HPLC was performed on a Waters Autopurification ZQ System equipped with a 2767 Sample Manager, a 2525 Binary Gradient Module and a 2996 Photodiode Array Detector, coupled to Waters Micromass ZQ analyzer. The HPLC purifications were performed on XTerra Prep RP C18 (19 x 150 mm) columns, using reverse-phase conditions (2 to 100% acetonitrile with 0.1% TFA over 20 min). X-ray Photoelectron Spectroscopy (XPS) data were collected by Axis Ultra and Axis Nova instruments (Kratos analytical, Manchester, UK) under ultra-high vacuum condition (<10-8 Torr) and using a monochromatic A1 *K*α X-ray source (1486.6 eV).

The source power was maintained at 225 or 150 W and the emitted photoelectrons were sampled from a 750 µm X 300 µm area. The analyzer pass energy was 160 or 80 eV for survey spectra and 40 eV for high-resolution spectra. The adventitious carbon Is peak was calibrated at 285 eV and used as an internal standard to compensate for any charging effects. MS: Nermag R-10-10C, chemical ionization (NH3) mode m/z (amu) [% relative base peak (100%)]. Semi-preparative HPLC: XTerra Prep RP C18 (19 x 150).

### Example 1: Synthesis of exemplary linker in accordance with the invention

A linker containing the required moieties for the biomaterial attachment, covalent binding of the human fetal osteoblasts by click reaction and the adhesion of the endothelial cells by ligand-protein recognition is synthezised. The synthesis of the final linker was presented in the scheme shown in **Figure 1****.** In the present examples, the numbers used to refer to specific compound correspond to those shown in Figure 1.

In a first synthetic step, the N-Boc-azidonorleucine was coupled to a mono-protected diaminoalkane in presence of PyBOP as coupling reagent. After deprotection of the amine under basic conditions, the gallic acid was added under the same coupling conditions. After cleavage of the Boc protecting group under acidic conditions, the intermediate **18** reacted with RGD-derivative **19** by click reaction in presence of a copper catalyst. Finally the activated DIBO was coupled to compound **20,** which was deprotected under basic conditions to afford the final linker **21.**

### Example 1.a Synthesis of intermediate (15)

### N-(4-aminobutyl)-6-azido-N²-(tert-butoxycarbonyl)-D-norleucinamide

N-Boc-azidonorleucine (0.3 g, 0.7 mmol, 1 equiv) was dissolved in dry DCM (15 mL). PyBOP (0.34 g, 0.8 mmol, 1.2 equiv) and diisopropylethylamine (0.3 mL, 2.0 mmol, 3 equiv) were added and the reaction mixture was stirred for 15 min at rt. Finally Fmoc-aminobutanediamine (0.26 g, 0.7 mmol, 1 equiv) was added and the mixture was stirred overnight at rt. The product was concentrated under reduced pressure and purified by flash column chromatography (AcOEt/EP 1:1). The resulting product was dissolved in 20 % piperidine in DMF and stirred until total conversion of the starting material. The product was concentrated under reduced pressure and purified by flash column chromatography (DCM/MeOH 8:2 followed by CH₃CN/25 % NH₃ 5: 1) to give the desired compound (mass: 195 mg, yield: 86 % for 2 steps). IR (film): 3300, 2930, 2865, 2465, 2090, 1650, 1525, 1455, 1390, 1365, 1245, 1165, 1045, 1015, 860, 820, 640, 605, 545, 535, 525, 515, 505 cm-¹. ¹H NMR (400 MHz, MeOD): δ=3.97 (m, 1H, C**H**), 3.21 (m, 2H, C**H₂**), 2.70 (t, 2H, ²*J*= 6.6 Hz, C**H₂**), 1.75-1.48 (m, 12H, (C**H₂**)₆), 1.45 (s, 9H, **(CH₃**)₃).¹³C NMR (101 MHz, MeOD): δ=173.73 (C**q**), 156.41 (C**q**), 79.24 (C**q**), 54.66 (**C**H), 50.28 (**C**H₂), 40.24 (**C**H₂), 38.55 (**C**H₂), 31.66 (**C**H₂), 28.11 (**C**H₂), 27.37 (**C**H₃)₃, 26.24 (**C**H₂), 22.83 (**C**H₂). HRMS (ESI): (*m*/*z*): calcd for C₁₅H₃₀N₆O₃+H: 343.2458, found: 343.2454. [α]_{D}²⁵ = -82 (c=0.005, MeOH).

### Example 1.b Synthesis of intermediate (17)

### 5-({4-[(6-azido-D-norleucyl)amino]butyl}carbamoyl)benzene-1,2,3-triyl triacetate

Acetoxygallic acid (61 mg, 0.2 mmol, 1 equiv) was dissolved in dry DCM (1 mL) in presence of PyBOP (125 mg, 0.3 mmol, 1.2 equiv) and stirred for 15 min at rt. Then a second solution containing *N*-(4-aminobutyl)-6-azido-*N*²-(*tert*-butoxycarbonyl)-D-norleucinamide (70 mg, 0.2 mmol, 1 equiv) and diisopropylethylamine (51 µl, 0.3 mmol, 1.5 equiv) in dry DCM (1 mL) was added and the reaction mixture was stirred overnight at rt. The product was concentrated under reduced pressure and purified by flash column chromatography (AcOEt/EP 3 : 1). The product was then dissolved into a solution of 4N HCl in dioxane (2 mL). The reaction mixture was stirred for 30 min and then concentrated under reduced pressure. HCl was coevaporated three times with Et₂O (mass : 44 mg, yield : 43 % for 2 steps). IR (film): 3265, 2935, 2865, 2380, 2095, 1775, 1660, 1585, 1490, 1435, 1370, 1320, 1180, 1115, 1050, 1010, 975, 895, 845, 780, 745, 700, 640, 615, 590, 555, 535 cm-¹.¹H NMR (400 MHz, MeOD): δ=7.65 (s, 2H, aromatics) 3.84 (m, 1H, C**H**), 3.39 (t, 2H, ²*J*= 6.6 Hz, C**H₂**), 3.27 (m, 2H, C**H₂**), 2.30 (s, 9H, (C**H₃**)₃), 1.88-1.45 (m, 12H, (C**H₂**)₆). ¹³C NMR (101 MHz, MeOD): δ=168.65 (C**q**), 168.33 (C**q**), 167.15 (C**q**), 143.54 (C (aromatic)), 127.11 (C (aromatic)), 126.43 (C (aromatic)), 119.77 (C (aromatic)), 53.07 (**C**H), 50.63 (**C**H₂), 39.32 (**C**H₂), 38.85 (**C**H₂), 30.80 (**C**H₂), 28.03 (**C**H₂), 26.31 (**C**H₂), 26.19 (**C**H₂), 21.77 (**C**H₂), 19.15 (**C**H₃)₃. HRMS (ESI): (*m*/*z*): calcd for C₂₃H₃₂N₆O₈+H: 521.2360, found: 521.2373. [α]_{D}²⁵ = -73 (c=0.004, MeOH).

### Example 1.c Synthesis of intermediate (19)

### Cyclo(L-arginylglycyl-L-α-aspartyl-D-phenylalanyl-N⁶-pent-4-ynoyl-L-lysyl)

4-pentynoic acid (1 g, 10 mmol, 1 equiv), was dissolved in DMF (20 mL) in presence of N-hydroxysuccinimide (1.77 g, 15 mmol, 1.5 equiv) and EDCI (2.93, 15 mmol, 1.5 equiv). The reaction mixture was stirred overnight at rt, concentrated under reduced pressure and finally purified by flash column chromatography (EP/AcOEt 3:2 then 1:1) to give the desired intermediate (mass: 1.67 g, yield: 84 %). 1-(pent-4-ynoyloxy)pyrrolidine-2,5-dione (74 mg, 0.38 mmol, 1 equiv) and *Cyclo*(Arg(Pbf)-Gly-Asp(OtBu)-D-Phe-Lys) (230 mg, 0.38 mmol, 1 equiv) were dissolved in DMF. Triethylamine (79 µl, 0.57 mmol, 1.5 equiv) was added and the reaction mixture was stirred until total conversion of the starting material. The product was concentrated under reduced pressure, dissolved in MeOH followed by precipitation by slow adding of diethyl ether to afford the pure product (mass : 181 mg, yield : 70 %). IR (film): 3265, 2935, 1635, 1540, 1435, 1370, 1200, 1080, 840, 650, 600 cm-¹. HRMS (ESI): (*m*/*z*): calcd for C₃₂H₄₅N₉O₈+H: 684.3469, found: 684.3489. [α]_{D}²⁵ = -4.8 (c=0.001, MeOH).

### Example 1.d Synthesis of intermediate (20)

*[(2S,5R,8S,11S)-8-[4-({3-[1-(5-amino-6-oxo-6-{[4-({[3,4,5-tris(acetyloxy)phenyl]carbonyl} amino)butyl]amino}hexyl)-1H-1,2,3-triazol-4-yl]propanoyl}amino)butyl]-5-benzyl-11-(3-carbamimidamidopropyl)-3,6,9,12,15-pentaoxo-1,4,7,10,13-pentaazacyclopentadecan-2-yl] acetic acid*

Cyclo(L-arginylglycyl-L-α-aspartyl-D-phenylalanyl-*N*⁶-pent-4-ynoyl-L-lysyl) and 5-({4-[(6-azido-D-norleucyl)amino]butyl}carbamoyl)benzene-1,2,3-triyl triacetate were dissolved in a mixture of H₂O/DMF (3 :1, 180:60 µL) in presence of a catalytic amount of CuSO₄ and sodium ascorbate. The reaction mixture was ultrasonicated for 6 h and then concentrated under reduced pressure. The crude product was used without further purification (quantitative yield). IR (film): 3275, 2930, 1770, 1635, 1520, 1435, 1320 1180, 1095, 1050, 845, 650, 605 cm-¹ HRMS (ESI): (*m*/*z*): calcd for C₅₅H₇₇N₁₅O₁₆+H: 1204.5751, found: 1204.5715. [α]_{D}²⁵= -26 (c=0.001, MeOH).

### Example 1.e Synthesis of linker compound (21)

*{[(2S,5R,8S,11S)-5-benzyl-11-(3-carbamimidamidopropyl)-8-[4-({3-[1-(5-{[11, 12-didehydro-5,6-dihydrodibenzo[a,e] [8] annulen-5-yloxy) carbonyl]amino}-6-oxo-6-[(4-{[(3,4,5-trihydroxyphenyl)carbonyl]amino}butyl)amino]hexyl)-1H-1,2,3-triazol-4-yl] propanoyl}amino)butyl]-3,6,9,12,15-pentaoxo-1,4,7,10,13-pentaazacyclopentadecan-2-yl} acetic acid*

[(2S,5R,8S,11S)-8-[4-({3-[1-(5-amino-6-oxo-6-{[4-({[3,4,5-tris(acetyloxy)phenyl]carbonyl}amino)butyl]amino}hexyl)-1*H*-1,2,3-triazol-4-yl]propanoyl}amino)butyl]-5-benzyl-11-(3-carbamimidamidopropyl)-3,6,9,12,15-pentaoxo-1,4,7,10,13-pentaazacyclopentadecan-2-yl] acetic acid (52 mg, 0.04 mmol, 1 equiv) was dissolved in dry DMF, carbonic acid, 5,6-dihydro-11,12-didehydro-dibenzo[*a*, *e*]cycloocten-5-yl ester, 4-nitrophenyl ester (17 mg, 0.04 mmol, 1 equiv) followed by triethylamine (18 µL, 0.13 mmol, 3 equiv) were added and the reaction mixture was stirred overnight at rt. The product was concentrated under reduced pressure and purified by precipitation in DCM (mass 35 mg, yield: 56 %). The resulting product was dissolved in a mixture of THF/DMF (1:1) in presence of a 1 M LiOH solution (72 µL, 0.07 mmol, 3 equiv) and ultrasonicated for 3 h until total deprotection of the starting material. The product was purified by HPLC ((XTerra Prep RP C18, (25x150 mm Waters)) to give the final product (masse: 5 mg, yield: 15 %). IR (film): 3670, 3020, 2170, 2160, 1730, 1490, 1435, 1366, 1231, 1015, 950, 875, 655, 600 cm⁻¹. HRMS (ESI): (*m*/*z*): calcd for C₆₆H₈₁N₁₅O₁₅+H: 662.8096, found: 662.8085. [α]_{D}²⁵ = -8.3 (c=0.001, DMSO).

### Example 2: Preparation of alumina-based bioceramics

Open porous, alumina scaffold materials are produced as described in F. Krauss Juillerat et al. J. Am. Ceram. Soc. 2011, 94, 77-83.

Calcium aluminate is used as setting agent in order to solidify the wet foams and stop bubble coarsening. Foam microstructure was determined by controlling of the bubble growth in the wet stage. Particle concentration in suspension is the most suitable parameter for inducing and controlling bubble growth. Three different scaffolds S1-S3 are produced, with scaffolds 1 and 2 having average pore diameters of 170 µm and an average window size of 50 to 60 µm. Scaffold 3 had a average pore diameter of 460 µm and windows of almost 100 µm.

### Example 3: Biocompatibility of ceramic foams

Scaffolds S1-S3 produced in Example 2 are all immersed in cell culture medium containing different cell types (human fetal osteoblasts, human U₂Oₛ, SaOs osteosarcoma cells) and cultured during 37 days. It is found that the different foam microstructures influence the migratory potential of the cells but not the cell viability, permitting either localized (S 1 and S2) or widespread (S3) colonization of the scaffold. This demonstrated that scaffolds with larger average pore sizes (S3) are more permissive for the trans-scaffold passage of cells across the entire structure than the scaffolds showing smaller average pore sizes. Furthermore, the cell viability assays also showed that the chemical composition did not influence the cell response to the different scaffolds. The cell-loaded and incubated scaffolds as prepared by the team of MER Dr Lucienne Juillerat, CHUV where analyzed by means of SEM. To do so, the foam discs where broken in half and the cross-sections scanned for cells. It could be shown that cells where found throughout the entire scaffold cross-sections for all combinations of cells (human fetal osteoblasts, human U₂Os, SaOs osteosarcoma cells) and scaffold types (S1-S3). Hence, the interconnecting pore networks of all three scaffold types are permissive for cell diffusion and suitable for cell colonization. For example, human fetal osteoblasts are spread out on the scaffold surface of S3 and/or spanning over pore interconnections, showing focal adhesion. These results are indicating a good acceptance of the living cells towards the scaffold material.

### Example 4: Ligation of linker compound (21) to the bioceramics of Example 2

Ceramic scaffold S1-S3 of Example 2 are immersed in a 1 nm aqueous solution of compound 21 at room temperature for 16 hours, in accordance with the procedure disclosed in H. Comas et al, ACS Appl. Mater. Interfaces 2012, 4, 573-573 (Supporting Information). In this way, functionalized biomaterials are obtained.

### Example 5: Seeding of functionalized biomaterials with human fetal osteoblasts

The functionalized biomaterials of Example 4 were seeded with human fetal osteoblasts as disclosed in Borcard, F.; Krauss Juillerat, F.; Staedler, D., Scaletta, C.; Applegate. L. A.; Comas, H.; Gauckler, L. J. ,Gerber-Lemaire, S; Juillerat-Jeanneret, L.; Gonzenbach, U. Bioconjugate Chem., 2012, 23, 2278-2290.

### Example 6: Synthesis of a further linker compound

Using the same general experimental procedure as set out in Example 1 (Figure 1), a compound is synthesized in which the cyclic RGD peptide is replaced by a IKVAV peptide (SEQ ID NO: 3) of laminin. Instead of intermediate 19 in Fig. 1, the intermediate of formula (V) below is used: 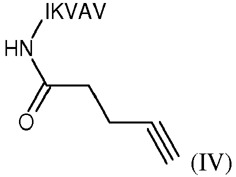 in which the peptide bond in formula (IV) is formed with the amino group at the ε carbon in the side chain of the lysine moiety of the IKVAV peptide (SEQ ID NO: 3).

## Claims

1. A compound of formula (I) below: 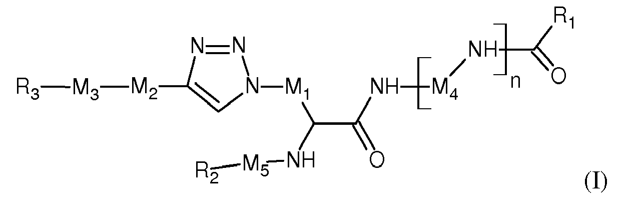 wherein:
M₁, M₂ and M₄ are, independently, selected from organic moieties consisting of 1 to 21 carbon atoms and from 0 to 15 heteroatoms;
R₃ is selected from a substituent comprising a peptide, saccharide, peptidoglycan, glycopeptide, and/or analogues of any one of the aforementioned;
M₃ is selected from:
-NH-CO- if R₃ comprises a peptide, peptidoglycan or glycopeptide substituent, bound by way of amide bond M₃ formed with a free amino group of said peptide, peptidoglycan or glycopeptide, respectively; and
-O-, if R₃ comprises a saccharide, a peptidoglycan or a glycopeptide substituent, bound by way of a ether bond formed with a primary hydroxyl group of a monosaccharide moiety of said saccharide, peptidoglycan or glycopeptide, respectively;
n is 0 or an integer from 1 to 3;
R₁ is an organic substituent comprising from 1 to 20 carbon atoms and 1 to 10 heteroatoms,
said organic substituent comprising at least one anchoring group;
M₅ is selected from moieties of formulae (1) and (2) below: 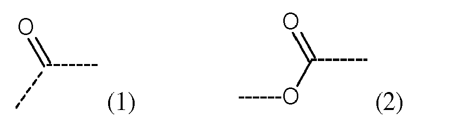 wherein
in the moieties of formulae (1) and (2) the dotted line on the right represents the bond by which M₅ is bound to the -NH- moiety in the structure of formula (I) and the dotted line on the left represents the bond to R₂;
R₂ is selected from any one of substituents (i) to (iv) below:
i. organic substituents comprising from 3 to 35 carbons and from 0 to 15 heteroatoms and comprising at least one carbon-carbon triple bond;
ii. a substituent of formula (3) below: 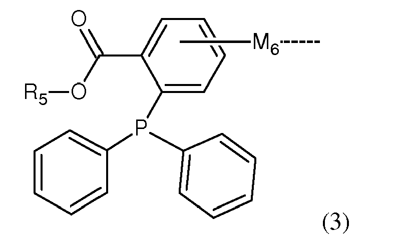 wherein the dotted line represents the bond by which R₂ is connected to the -CO- or -O-CO- group of the moiety of formulae (1) or (2), respectively;
M₆ is an organic moiety consisting of 1 to 30 carbon atoms and 0 to 20 heteroatoms; and R₅ is an organic substituent comprising from 1 to 20 carbons and from 0 to 10 heteroatoms;
iii. a substituent of formula (4) below: 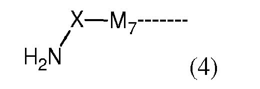 wherein -X- is selected from -NH- and from -O-, and M₇ is an organic moiety comprising from 1 to 10 carbon atoms and 0 to 5 heteroatoms; and,
iv. a substituent of formula (5) below: 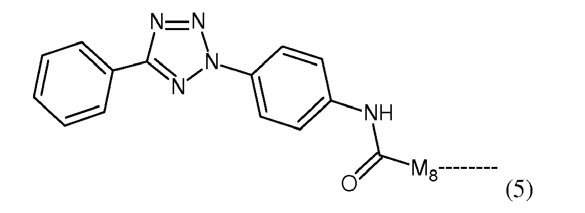 wherein M₈ is an organic moiety comprising from 1 to 10 carbon atoms and from 0 to 5 heteroatoms;
any one or more of the hydrogens of the phenyl moiety and/or the phenyl substituent in the structure of formula (5) may be substituted by halogen or C1-C8 linear, branched and/or cyclic alkyl, preferably C1-C4, linear, branched and/or cyclic alkyl, and most preferably said hydrogens are not substituted or may be substituented totally or partially by halogen.

2. The compound of claim 1, wherein said substituent (i) (R₂) comprising from 3 to 35 carbons and from 0 to 15 heteroatoms and comprising at least one carbon-carbon triple bond comprises a structure of formula (6) below: 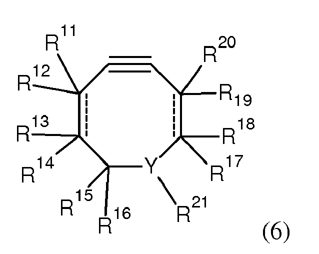 wherein:
Y is N or CR²², R²² being selected, independently, as defined for substituents R¹¹ to R²¹, with the proviso that if Y is N, R²¹ is selected from H and substituents -CH₂-R²²;
R¹¹ to R²¹ are independently selected from H, halogen, -OH, -NH₂, and organic substituents comprising from 1 to 20 carbon atoms and 0 to 15 heteroatoms, wherein, a substituent pair of substituents on the same ring carbon (for example, R¹¹ and R¹²) can form any one selected from: an =O (oxo group), a carbon-carbon double bond, such as =CR^{11'}R^{12'}, in case of R¹¹ and R¹², and a substituent comprising a delocalized π-bond; wherein R^{11'} and R^{12'}, and so forth, as applicable, are independently selected from H, halogen, and from organic substituents comprising from 1 to 19 carbon atoms and 0 to 15 heteroatoms, wherein substituents on neighbouring ring carbons, including a substituent =CR^{11'}R^{12'} and so forth, as applicable, with one or two substituents on a neighbouring ring carbon, may form a ring fused to the 8-atom ring in formula (6);
wherein said combined dotted and solid lines in the 8-atom ring each represent, independently, a single bond, a single bond and a delocalized π-bond, or a double bond, wherein, if any one or both of said dotted and solid lines are double bonds, the substituents R¹² and R¹⁴, and/or R¹⁸ and R²⁰, respectively, are absent, so as to observe to an applicable valence, and wherein, if any one or both of said dotted and solid lines contain delocalized π-bonds, at least one of the substituent pairs R¹¹/R¹² and R¹³/R¹⁴, and/or, respectively, R¹⁷/R¹⁸ and R¹⁹/R²⁰, also contain delocalized π-bonds and substituent pairs on neighbouring ring carbons may be connected with each other to form an aromatic system fused to the 8-atom ring of formula (6); and,
wherein at least one of said substituents R¹¹ to R²¹ and R²² is either a moiety of formula (7) below:
-------M₉------- (7), or directly connects the structure of formula (6) by way of single bond of a ring carbon of the 8-atom ring or of a -CH₂- moiety in said structure (in case of -CH₂-R²²), to the carbon or oxygen, respectively, in the moieties of formulae (1) and (2);
wherein M₉ is an organic moiety comprising from 1 to 10 carbon atoms and from 0 to 5 heteroatoms, and the dotted lines connected on the left and right sides to M₉ represent the bonds by which M₉ is connected to the structure of formula (6) and to the substituent of formula (1) or (2).

3. The compound of claim 1 or claim 2, wherein said substituent (i) (R₂) comprising from 3 to 35 carbons and from 0 to 15 heteroatoms and comprising at least one carbon-carbon triple bond comprises and/or consists of any one selected of the structures of formulae (8) to (12) below: 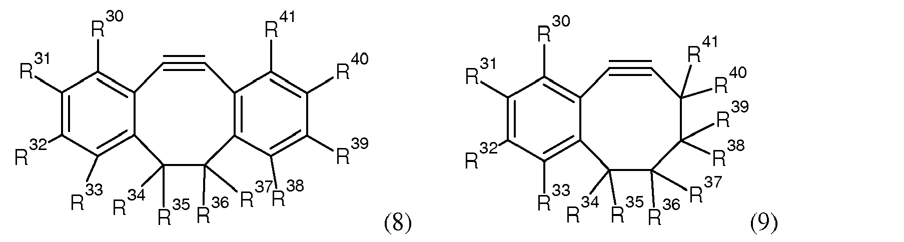 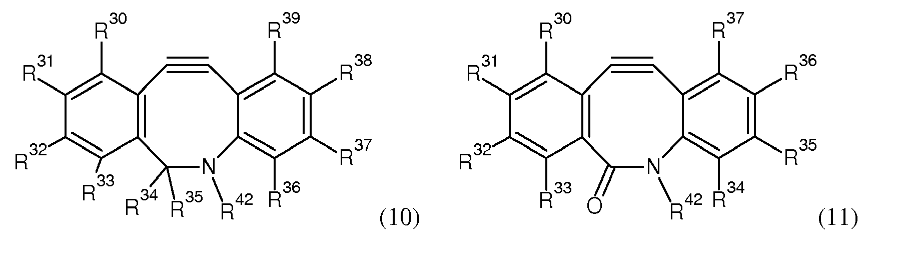 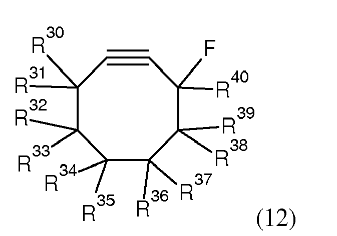 wherein R³⁰ to R⁴¹, in as far as present, are independently selected from H, halogen, -OH, - NH₂, and organic substituents comprising from 1 to 10 carbon atoms and 0 to 5 heteroatoms and wherein a pair of substituents on the same ring carbon may form an oxo group (=O); wherein R⁴² is selected from H and substituents -CH₂-R⁴³, R⁴³ being selected, independently, as defined for substituents R³⁰⁻⁴¹; and,
wherein at least one of said substituents R³⁰⁻⁴¹; R³⁰⁻⁴¹; R³⁰⁻³⁹ and R⁴³; R³⁰⁻³⁷ and R⁴³; and R³⁰⁻⁴⁰ in formulae (8); (9); (10); (11); and (12), respectively, is either a linker moiety of formula (7) below:
-------M₉------- (7), or directly connects the structure of formula (8), (9), (10), (11) or (12), by way of single bond of a ring carbon or of a -CH₂- moiety in said structures, to the carbon or oxygen atom, respectively, in the moieties of formulae (1) and (2);
wherein, in the moiety of formula (7), M₉ is an organic moiety comprising from 1 to 15 carbon atoms and from 0 to 10 heteroatoms, and the dotted lines connected on the left and right sides to M₉ represent the bonds by which M₉ is connected to one of the structures of any one of formulae (8) to (12) and, on the other hand, to the moiety of formula (1) or (2), respectively.

4. The compound of any one of claims 1 to 3, wherein said substituent (i) (R₂) comprises any one selected from the structures of formulae (13) to (18) below: 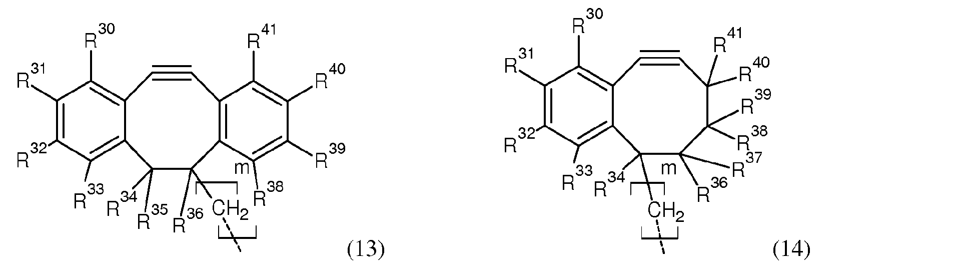 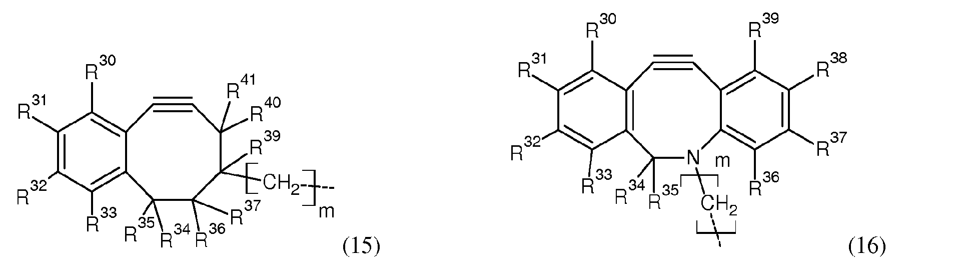 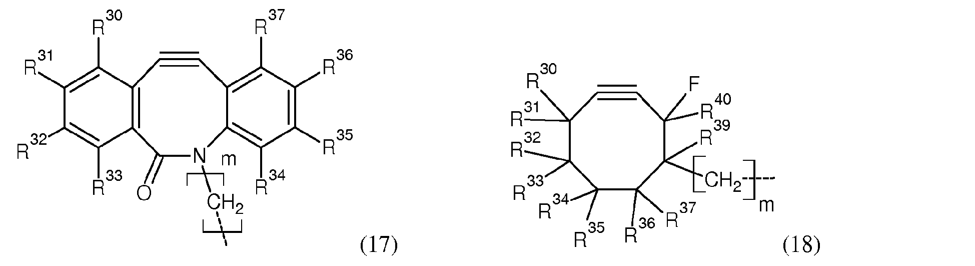 wherein
m is an integer of 1 to 5 and, in formulae (13), (14), (15), and (18) may also be 0 (zero), and
R³⁰ to R⁴¹, in as far as present, are independently selected from H, halogen, -OH, -NH₂, and organic substituents comprising from 1 to 10 carbon atoms and 0 to 5 heteroatoms, wherein, a pair of substituents on the same ring carbon may form an oxo group (=O), and wherein the dotted line represents the bond by which the structure of any one of formulae (13) to (18) is connected to the moiety of formula (1) or (2).

5. The compound of any one of the preceding claims, wherein M₁, M₂, M₄ and M₉ are, independently, selected from (-CH₂)ᵣ-, (-CH₂-CH₂-O)ᵣ-CH₂- and -CH₂(-O-CH₂-CH₂)ᵣ-, and M₁ and M₂ are further selected from (-CH₂-CH₂-O)ᵣ, wherein r is, independently for M₁, M₂ and M₄, an integer of 1 to 10.

6. The compound of any one of the preceding claims, wherein R₃ comprises a peptide substituent selected from the group consisting of: an RGD peptide, a cadherin, a catenin, an integrin, collagen IV, an elastin, a fibronectin, a laminin, a peptide sequence of at least three amino acid moieties taken from any one of the aforementioned peptides/proteins, and analogues of any one of the aforementioned.

7. The compound of any one of the preceding claims, wherein R₁ is a substituent of formulae (20) below: 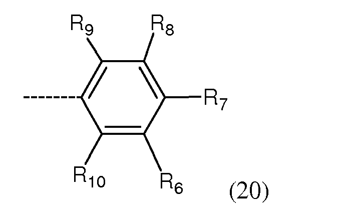 wherein:
R₆ to R₁₀ are independently selected from H, halogens and from C1-C10 substituents comprising from 0 to 5 heteroatoms, with the proviso that at least one of R₆ to R₁₀ comprises or consists of an anchoring group, preferably selected from-OH and -COOH.

8. The compound of any one of the preceding claims, wherein said substituent R₁ is selected from substituents of formulae (21) and (22) below:

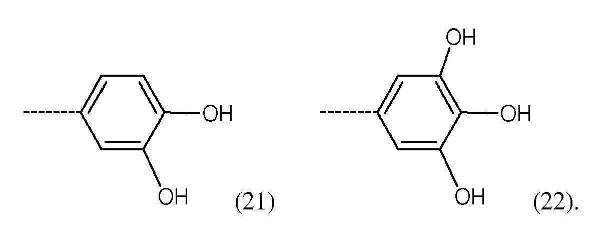

9. The compound of any one of the preceding claims, wherein M₃ is -NH-CO-, and R₃ is a RGD-peptide substituent of formula (30) below: 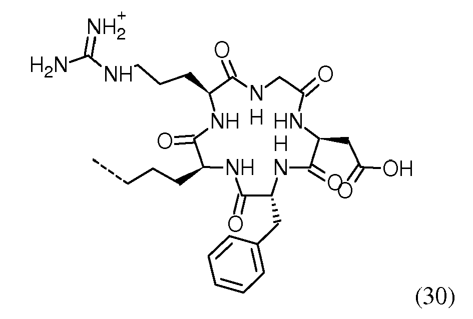 wherein the dotted line on the left in formula (30) represents the single bond by which R₃ is connected to the -NH- moiety of M₃.

10. The compound of any one of the preceding claims, which is a compound of any one of formulae (II), (IIa), (III) and (IIIa) below: 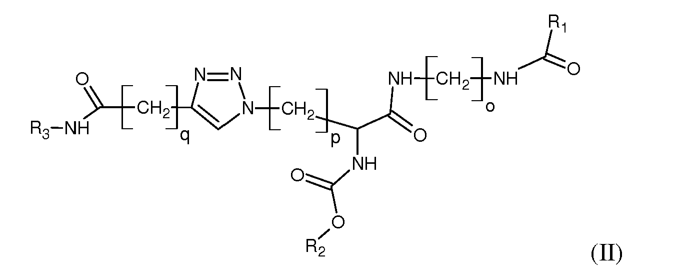 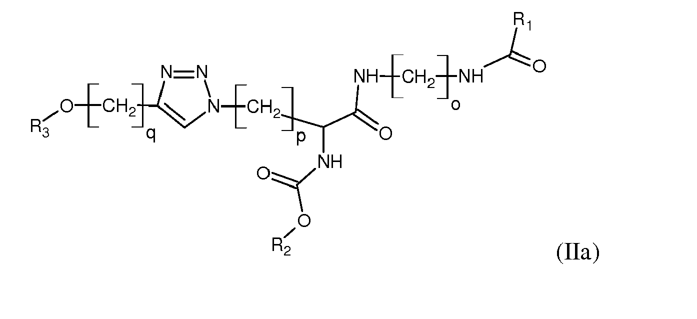 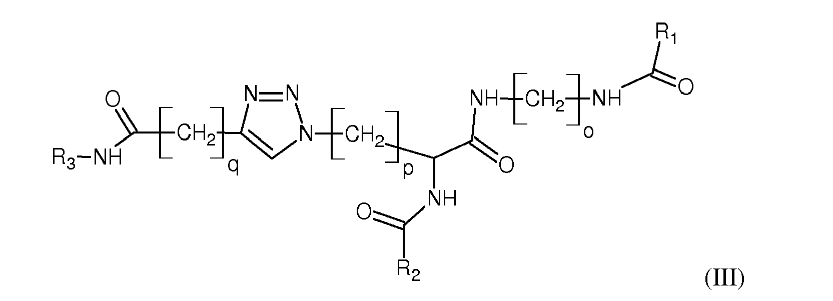 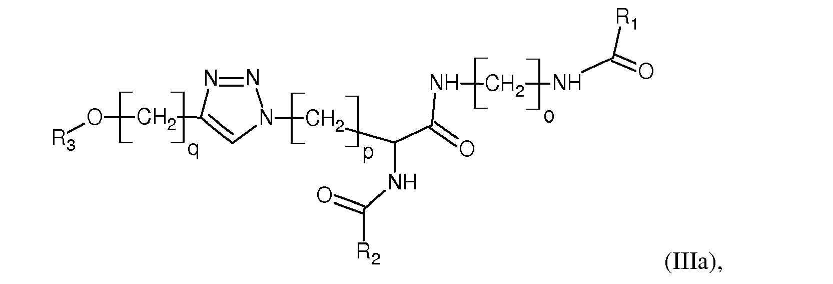 wherein o, p and q are, independently, selected from an integer of 1 to 10; and R₁, R₂ and R₃ are as defined in any one of the preceding claims.

11. A compound comprising a structure of formula (IV) below:

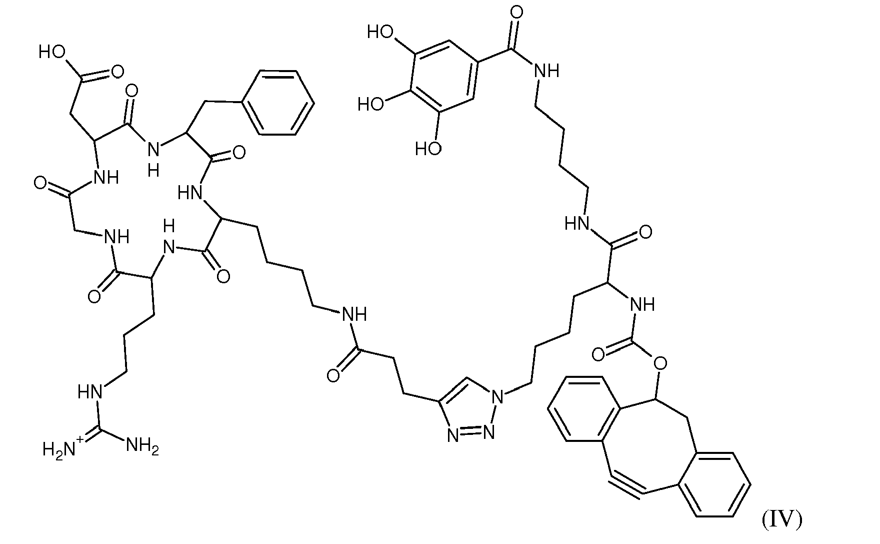

12. An implant material wherein a compound of any one of claims 1 to 11 is bound to said implant material.

13. The implant material of claim 12, wherein said implant material comprises a polymer and/or a ceramic selected from the group consisting of alumina, hydroxyapatite and tricalcium phosphate, and a combination comprising two or more of these materials.

14. The implant material of claim 12 or claim 13, which comprises a porous polymer, a ceramic foam, a porous ceramic material and/or a combination comprising two or more of the aforementioned.

15. Use of a compound of formula (XX) in the synthesis of an organic linker suitable to functionalize an implant material, 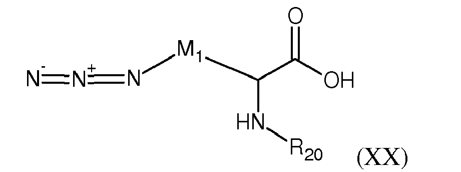 wherein R₂₀ is H or a protective group; and
M₁ is an organic moieties comprising from 1 to 21 carbon atoms and from 0 to 15 heteroatoms.


SEQUENCE LISTING

<110> Ecole Polytechnique FÃ©dÃ©rale de Lausanne (EPFL)

<120> Compounds for Functionalizing Biomaterials

<130> P-0091-EP

<160> 3

<170> BiSSAP 1.0

<210> 1
<211> 15
<212> PRT
<213> Homo sapiens

<220>
<221> SOURCE
<222> 1..15
<223> /mol_type="protein"
      /note="Sequence stretch of Collagen IV"
      /organism="Homo sapiens"

<400> 1
Gly Val Lys Gly Asp Lys Gly Asn Pro Gly Trp Pro Gly Ala Pro
1               5                   10                  15

<210> 2
<211> 5
<212> PRT
<213> Homo sapiens

<220>
<221> SOURCE
<222> 1..5
<223> /mol_type="protein"
      /note="Sequence stretch 1 of laminin"
      /organism="Homo sapiens"

<400> 2
Tyr Ile Gly Ser Arg
1               5

<210> 3
<211> 5
<212> PRT
<213> Homo sapiens

<220>
<221> SOURCE
<222> 1..5
<223> /mol_type="protein"
      /note="Sequence stretch 2 of laminin"
      /organism="Homo sapiens"

<400> 3
Ile Lys Val Ala Val
1               5